# EUROPEAN PATENT APPLICATION

(11) **EP 4 703 002 A1**
(43) Date of publication of application: **04.03.2026**
(21) Application number: 25198307.8
(22) Date of filing: 27.08.2025
(51) Int. Cl.: A61N 1/06, A61N 1/32

(54) **TREATMENT TIPS FOR HANDPIECE**

(30) Priority: 27.08.2024 KR 20240114776
(71) Applicant: VIOL Co. Ltd., Bundang-gu Seongnam-si, Gyeonggi-do, 13510 (KR)
(72) Inventor: KIM, Jongkil, 13510 Seongnam-si, Gyeonggi-do (KR); LEE, Sangjin, 13510 Seongnam-si, Gyeonggi-do (KR)
(74) Representative: Zimmermann, Tankred Klaus

(57) **Abstract**

Disclosed is a treatment tip for a handpiece of an invasive RF treatment device. The treatment tip for a handpiece includes a needle part including a plurality of microneedles and a guide part including a plurality of tubular needle guides in accordance with the plurality of microneedles. The plurality of tubular needle guides is disposed in one or more lines. The plurality of microneedles is disposed within the plurality of tubular needle guides. A virtual line that connects the needle entry and exit ends of the plurality of tubular needle guides disposed in one line has curvature.

## Description

### [Technical Field]

The present disclosure relates to a treatment tip for a handpiece and, particularly, to a treatment tip for a handpiece of an invasive radio frequency (RF) treatment device, which treat a scalp based on the principle that electric stimuli (e.g., RF stimuli) are applied by penetrating a microneedle into the scalp.

### [Background Art]

One of skin treatment methods which are recently in the spotlight is microneedle treatment. The microneedle treatment is a method of maximizing the natural healing and regeneration of the skin, by forming a treatment pillar by penetrating a very small needle into the skin and simultaneously creating fine scars for facilitating the regeneration of the skin by applying a radio frequency (RF) and inducing the growth of cells as the fine scars are healed up.

When the RF is applied to human tissues through the microneedle that plays a role as an electrode for providing RF stimuli, collagen components are firmly united in the dermal layer and the forming of new collagen is facilitated because friction heat attributable to the RF is generated within skin tissues around the microneedle. Accordingly, skin improvement effects, such as the stretching of wrinkles and a reduction in the pores of the skin, can be achieved.

The microneedle treatment can regenerate collagen and elastic fiber by generating heat (about 40 to 60°C) in a target portion while not causing burn in epidermis. For this reason, the microneedle treatment has been known to be very effective in acne, pimple scars, fine wrinkles, deep wrinkles, and pore reduction treatment. Furthermore, the microneedle treatment has been known to facilitate the combustion of a layer of fat by facilitating blood circulation and enabling the treatment of obesity by activating the activity of a lymphatic system.

In general, an invasive RF treatment device that enables the microneedle treatment includes a handpiece, a treatment tip, and a controller. Microneedles that function as electrodes are installed in the treatment tip in a way to move in a straight line. Accordingly, when the invasive RF treatment device operates with the treatment tip on a treatment portion, the microneedles simultaneously move and penetrate into the skin. In that state, a received RF signal applies electric stimuli to a corresponding skin layer.

The microneedle treatment can be performed on a scalp because the scalp is a kind of skin. When the microneedle treatment is applied to the scalp, the dermis tissues of the scalp can be newly constructed and rearranged, the rupture of hair follicles can be minimized, and the deformation or the scalp or the necrosis of cells can be prevented. Furthermore, effects in that collagen synthesis and scalp damage are recovered and wrinkles are stretched by stimulating the dermis of the scalp can be achieved.

However, it is difficult to secure the visibility of a treatment portion because the scalp is covered with a great deal of hair unlike other skin. Hair that has been pressed down in a process of bringing a treatment part of the invasive RF treatment device into contact with the scalp for a treatment makes the treatment difficult by hindering the penetration of the microneedle into the scalp, or pains may be caused when the microneedle penetrates into the scalp because the microneedle is deformed by the pressed hair.

Furthermore, most of conventional invasive RF treatment devices each have needle arrangement in which the front ends of microneedles that protrude to the outside for treatment are arranged on the same plane. Unlike another body portion, in the case of a scalp having curved contour, an insertion deviation (or a penetration depth deviation) may occur between the microneedles when treatment is performed. As a result, a treatment effect may be halved or a side effect may occur in severe cases because a skin layer having a desired depth is not accurately targeted.

### [Documents of Related Art]

### [Patent Document]

Korean Patent Application Publication No. 10-2015-0060312 (laid open on June 3, 2015)
Korean Patent Application Publication No. 10-2021-0055663 (laid open on May 17, 2021)

### [Summary of Disclosure]

### [Technical Problem]

An object of the present disclosure is to provide a treatment tip for a handpiece, which enable the visibility of a treatment portion to be smoothly secured when treatment is performed on a scalp and cab reduce the intervention of hair.

Another object of the present disclosure is to provide a treatment tip for a handpiece, which can solve an insertion deviation (or penetration depth deviation) problem between needles when treatment is performed on a scalp because the treatment tip has a geometrical shape suitable for scalp treatment.

### [Solution to Problem]

According to an aspect of the present disclosure, there is provided a treatment tip for a handpiece of an invasive radio frequency (RF) treatment device, including a needle part including a plurality of microneedles and a guide part including a plurality of tubular needle guides in accordance with the plurality of microneedles. The plurality of tubular needle guides is disposed in one or more lines. The plurality of microneedles is disposed within the plurality of tubular needle guides. A virtual line that connects the needle entry and exit ends of the plurality of tubular needle guides disposed in one line has curvature.

The treatment tip for a handpiece according to an embodiment of the present disclosure may further include a cap part detachably installed at a front end of the handpiece and an elevation pole having the needle part mounted thereon and movably disposed within the cap part.

In this case, the guide part may be disposed at a front end of the cap part.

In the treatment tip for a handpiece according to an embodiment of the present disclosure, the guide part may be integrally disposed at the front end of the cap part or may be disposed at the front end of the cap part in a form in which the guide part is detachable from the cap part.

In the treatment tip for a handpiece according to an embodiment of the present disclosure, among the plurality of tubular needle guides disposed in the one line, the lengths of the tubular needle guides disposed at the outermost parts on both sides thereof may be relatively long and the lengths of the tubular needle guide may be gradually reduced toward the center thereof.

In the treatment tip for a handpiece according to an embodiment of the present disclosure, two or more microneedles may be disposed within one tubular needle guide.

In this case, the two or more microneedles disposed within the one tubular needle guide may be formed to have an identical length.

In the treatment tip for a handpiece according to an embodiment of the present disclosure, upon drawing-out operation of the microneedles, the plurality of microneedles may be drawn out from the needle entry and exit ends of corresponding tubular needle guides in an identical length.

### [Advantageous Effects of Invention]

The treatment tip for scalp care according to an embodiment of the present disclosure has an advantage in that the treatment tip has a structure in which the microneedles can be safely penetrated into a scalp, that is, a structure suitable for scalp treatment because a unique component (i.e., the tubular needle guide) capable of avoiding or minimizing interference with surrounding hair upon treatment is applied while using an invasive type (i.e., a method of applying electric stimuli by penetrating the microneedles into a treatment target scalp) that is effective in scalp treatment.

Furthermore, the visibility of a treatment target portion for treatment can be smoothly secured through combing because the treatment tip has a hair comb shape having predetermined curvature. Furthermore, it is possible to greatly improve the accuracy and precision of treatment, such as significantly reducing an erroneous treatment or a side effect attributable to the erroneous treatment, because the microneedles can be accurately penetrated into a targeted a treatment target portion due to the smoothed securing of the visibility.

Furthermore, the front end of the guide part can stably come into contact with a scalp surface without being lifted off upon treatment because the guide part including the tubular needle guide has a geometrical shape corresponding to a shape of a scalp surface having curved contour. Furthermore, a conventional insertion deviation (or penetration depth deviation) between needles and a reduction of a treatment effect or a side effect problem attributable to the conventional insertion deviation can be clearly solved because the microneedles are drawn out from corresponding tubular needle guides in the same length in that state.

### [Brief Description of Drawings]

FIG. 1 is a diagram illustrating a form in which a treatment tip according to an embodiment of the present disclosure has been mounted at the front end of the handpiece of an invasive RF treatment device.
FIG. 2 is a diagram illustrating a form in which the treatment tip according to an embodiment of the present disclosure has been separated from the handpiece of the invasive RF treatment device.
FIG. 3 is a diagram illustrating the state in which the treatment tip for a handpiece according to an embodiment of the present disclosure has been combined.
FIG. 4 is a diagram illustrating that the treatment tip illustrated in FIG. 3 has been separated.
FIG. 5 is a cross-sectional diagram schematically illustrating internal components of the treatment tip illustrated in FIG. 3.
FIGS. 6A and 6B are cross-sectional views of a tubular needle guide for illustrating the state in which microneedles have been disposed.
FIGS. 7A to 7C are plan views of a guide part, which illustrates various arrangements of the tubular needle guide.
FIG. 8 is a diagram illustrating a preparation process for performing invasive RF treatment on a scalp by using the treatment tip according to an embodiment of the present disclosure.
FIG. 9 is a diagram illustrating a form in which invasive RF treatment is performed on a scalp by using the treatment tip according to an embodiment of the present disclosure.

### [Description of Embodiments]

Hereinafter, embodiments of the present disclosure are described in detail.

The embodiments are provided to more fully explain the present disclosure to a person having ordinary knowledge in the art to which the present disclosure pertains. The following embodiments may be modified in various other forms, and the scope of the present disclosure is not limited to the following embodiments. Rather, these embodiments are provided to make the present disclosure more thorough and complete and to fully convey the spirit of the present disclosure.

Terms used in this specification are used to describe a specific embodiment, and are not intended to limit the present disclosure. Furthermore, in this specification, an expression of the singular number may include an expression of the plural number unless clearly defined otherwise in the context.

In the present application, it is to be understood that a term, such as "include" or "have", is intended to designate that a characteristic, a number, a step, an operation, a component, a part or a combination of them described in the specification is present, and does not exclude the presence or addition possibility of one or more other characteristics, numbers, steps, operations, components, parts, or combinations of them in advance.

Furthermore, when it is said that one component is placed "ahead", "behind", "over", or "under" the other component, the one component may be disposed "ahead", "behind", "over", or "under" the other component by coming into contact with the other component, but it should also be understood that a third component is disposed between the one component and the other component unless specially described. Furthermore, when it is described that one component is "connected" to the other component, it should be understood that the one component is directly connected to the other component, but is indirectly connected to the other component.

The drawings are merely for enabling the spirit of the present disclosure to be understood, and it should not be interpreted that the scope of the present disclosure is limited by the drawings. Furthermore, in the drawings, a relative thickness or length or a relative size may be enlarged for convenience and the clarity of description.

A treatment tip according to an embodiment of the present disclosure is treatment tip for a handpiece of an invasive RF treatment device, which treats a scalp based on the principle that electric stimuli (RF stimuli) are applied by penetrating microneedles into the scalp. The treatment tip according to an embodiment can reduce and improve hair loss or black hair symptoms by inserting the microneedles into a scalp of a patient and transferring an RF through the inserted microneedles in order to improve an environment for the scalp.

Embodiments of the present disclosure are described in detail with reference to the accompanying drawings.

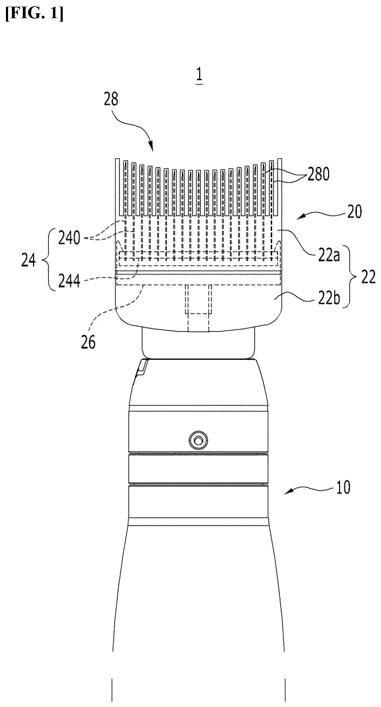

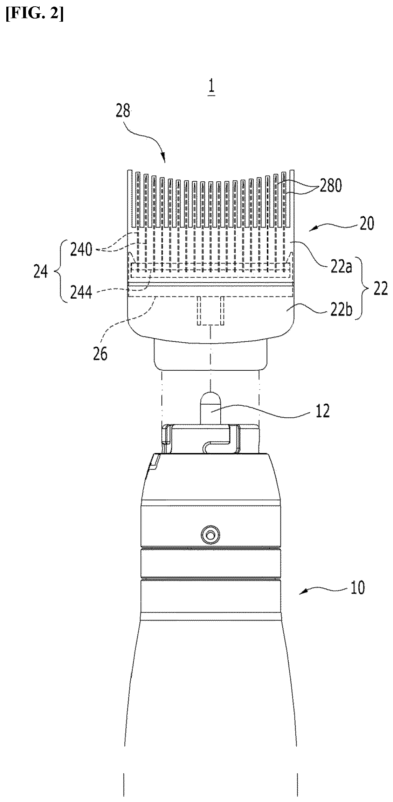

FIG. 1 is a diagram illustrating a form in which a treatment tip according to an embodiment of the present disclosure has been mounted at the front end of the handpiece of an invasive RF treatment device. FIG. 2 is a diagram illustrating a form in which the treatment tip according to an embodiment of the present disclosure has been separated from the handpiece of the invasive RF treatment device. A general construction of an invasive RF treatment device including a treatment tip according to an embodiment of the present disclosure is schematically described with reference to FIGS. 1 and 2.

Referring to FIGS. 1 to 2, the invasive RF treatment device 1 includes a handpiece 10. The invasive RF treatment device 1 further includes a treatment tip 20 for a handpiece according to an embodiment of the present disclosure, which is mounted at the front end of the handpiece 10. The treatment tip 20 may be detachably mounted at the front end of the handpiece 10 in a cartridge manner. Accordingly, the treatment tip 20 can be easily replaced from the handpiece 10, if necessary.

The treatment tip 20 includes a plurality of microneedles 240 to which an RF current is applied from the handpiece 10. The plurality of microneedles 240 may transfer the RF to a target skin tissue of a treatment subject by penetrating the target skin tissue. The treatment tip 20 including the plurality of microneedles 240 may be provided in various types in which the number of microneedles 240 or an arrangement form of the microneedles 240 is different depending on its use purpose. Accordingly, a surgical operator may perform a treatment task after mounting the treatment tip 20 suitable for treatment on the handpiece 10.

The handpiece 10 is connected to a main body (not illustrated) of the RF treatment device 1 by a cable, and may exchange signals or information with the main body. Although not illustrated in the drawing, the handpiece 10 may include a driving part that drives a needle part 24 within the treatment tip 20 in a straight line, an RF generation part that generates an RF and applies the RF to the needle part 24 including the plurality of microneedles 240, and a control part that controls the driving part and the RF generation part.

The driving part may move the needle part 24 including the plurality of microneedles 240 back and forth in a specific direction (e.g., up and down in the drawing) within a certain stoke range. The RF generation part may convert power supplied from the main body in the form of RF pulses and transfer the RF pulses to the needle part 24. Various forms, such as a motor, a ball screw, a pneumatic actuator, and an electromagnet actuator, may be applied to the driving part.

The handpiece 10 may include various manipulation buttons so that the various manipulation buttons are exposed to the outside. The control part may be electrically connected to the various manipulation buttons included in the handpiece 10. Accordingly, when a user manipulates a button of the handpiece 10, the control part may generate a corresponding control signal by recognizing the manipulation and transmit the corresponding control signal to the driving part and the RF generation part. Accordingly, operations of the driving part and the RF generation part may be controlled.

A driving shaft 12 may be connected to the driving part. A part of the driving shaft 12 may be exposed to the outside of the front end of the handpiece 10. The driving shaft 12 that is exposed to the front end of the handpiece 10 may be disposed within the treatment tip 20 and combined with an elevation pole 26 on which the needle part 24 is mounted. Accordingly, when the driving part operates, the elevation pole 26 combined with the driving shaft 12 and the needle part 24 mounted on the elevation pole 26 may be moved in a specific direction (e.g., up and down in the drawing).

The treatment tip 20 includes the needle part 24 and a cap part 22. The needle part 24 may include the plurality of microneedles 240 and a substrate 244 on which the plurality of microneedles 240 is mounted. The cap part 22 may form an appearance of the treatment tip 20, and may accommodate the needle part 24 in its internal accommodation space. The cap part 22 may be formed of an insulator, such as plastic, and may be detachably mounted at the front end of the handpiece 10.

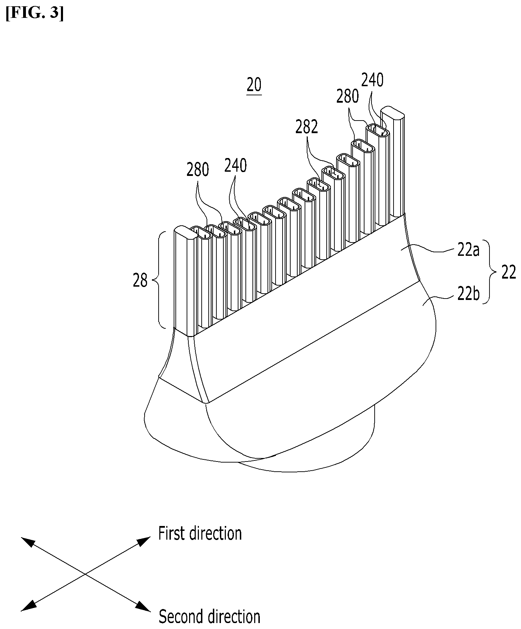

The components of the treatment tip for a handpiece according to an embodiment of the present disclosure are described more specifically with reference to FIGS. 3 to 5.

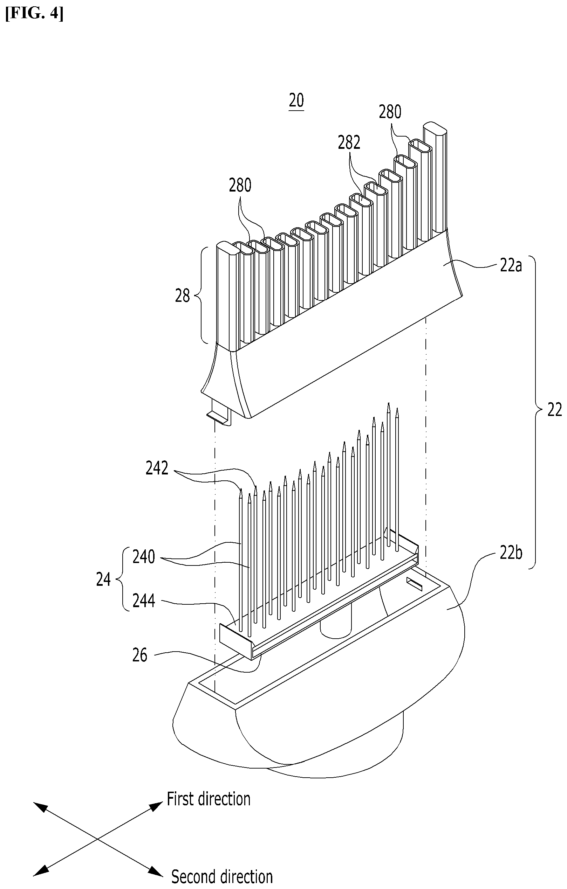

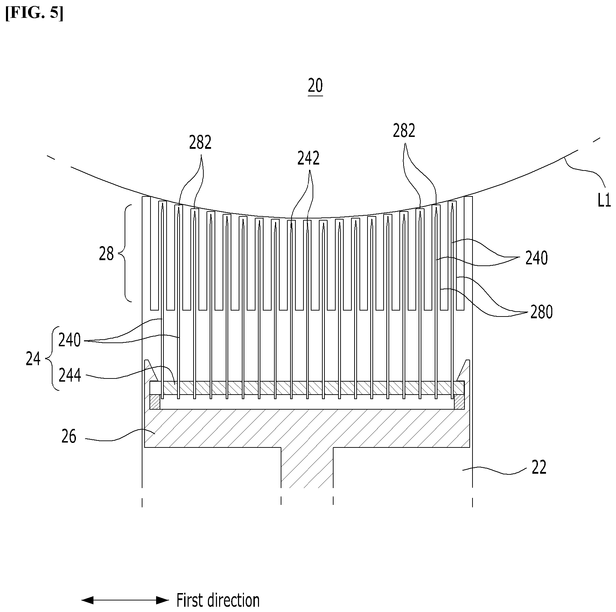

FIG. 3 is a diagram illustrating the state in which the treatment tip for a handpiece according to an embodiment of the present disclosure has been combined. FIG. 4 is a diagram illustrating that the treatment tip illustrated in FIG. 3 has been separated. Furthermore, FIG. 5 is a cross-sectional diagram schematically illustrating internal components of the treatment tip illustrated in FIG. 3.

Referring to FIGS. 3 to 5, the treatment tip 20 for a handpiece according to an embodiment includes the needle part 24 including the plurality of microneedles 240 and the cap part 22 that accommodates a part of the needle part 24. The needle part 24 includes the plurality of microneedles 240 and the substrate 244 on which all of the plurality of microneedles 240 are mounted. The cap part 22 is detachably mounted at the front end of the handpiece 10. Accordingly, the cap part 22, together with the needle part 24, can be separated from the handpiece 10, if necessary.

The elevation pole 26 may be disposed movably in a specific direction (e.g., up and down in the drawing) within the cap part 22. The elevation pole 26 is connected to the driving shaft 12 that protrudes from the handpiece 10. The substrate 244 of the needle part 24 may be combined with the elevation pole 26. Accordingly, when the driving part operates, the elevation pole 26 combined with the driving shaft 12 and the needle part 24 mounted on the elevation pole 26 may perform a relative motion in a specific direction (e.g., up and down in the drawing) with respect to the cap part 22 that is relatively a fixed body.

As in an example of FIG. 4, the cap part 22 may have a construction in which the cap part 22 is divided into an upper cap 22a and a lower cap 22b so that the upper cap 22a and the lower cap 22b can be separated and combined. In this case, the treatment tip 20 according to an embodiment may be completed by disposing the needle part 24 and the elevation pole 26 at a predetermined combination location in the state in which the upper cap 22a and the lower cap 22b have been separated from each other and then combining the upper cap 22a and the lower cap 22b. In this case, a snap joint may be used for the combination of the upper cap 22a and the lower cap 22b.

The plurality of microneedles 240 each functions as an electrode that transfers an RF generated by the RF generation part to a skin layer. The plurality of microneedles 240 may each protrude from the needle entry and exit end 282 of a guide part 28 in a predetermined length and penetrate into a target skin tissue by the relative motion of the needle part for the cap part in the specific direction, thus transferring the RF to the target skin tissue or returning to an initial location of treatment within the guide part 28.

The plurality of microneedles 240 may be basically divided into two groups. The microneedles 240 belonging to the same group may have the same polarity. The microneedles 240 belonging to different groups may have opposite polarities. For example, when microneedles belonging to one of the two groups are each a positive electrode (i.e., a + electrode), microneedles belonging to the other of the two groups are each a negative electrode (i.e., a - electrode). Accordingly, a (+) voltage may be applied to a positive electrode microneedle group, and a (-) voltage may be applied to a negative electrode microneedle group.

The front end of the cap part 22 may include the guide part 28. The guide part 28 may include a plurality of tubular needle guides 280 in accordance with the microneedles 240. The guide part 28 may be integrally constructed at the front end of the upper cap 22a along with the cap part 22 or may be provided at the front end of the upper cap 22a in a detachable self-assembly manner. The plurality of tubular needle guides 280 may each be formed in a hollow tube shape having various cross-sectional shapes, such as a square, a circle, and an oval.

A part of the microneedle 240 may be disposed within (i.e., a hollow part) the tubular needle guide 280, and another part of the microneedle 240 may be disposed within the cap part 22 along with the substrate 244. In a standby state for treatment, the apex 242 of the microneedle 240 is not exposed to the outside because the apex 242 of the microneedle 240 is fully inserted into a corresponding tubular needle guide 280. When the driving part operates in response to a treatment start command, the plurality of microneedles 240 may penetrate into a scalp by being simultaneously drawn out to the outside.

In the state in which a part (i.e., the apex 242) of the microneedle 240 drawn out from the tubular needle guide 280 has penetrated into the scalp, an RF current generated by the RF generation part is simultaneously applied to the plurality of microneedles 240. Accordingly, effects in that the activity of cells that constitute a dermis tissue within the scalp can be increased and collagen synthesis and scalp damage are recovered can be achieved because RF stimuli may be applied to an internal skin tissue.

The guide part 28 functions to provide guidance to a movement (i.e., a rectilinear motion) of the microneedle 240. Accordingly, upon RF treatment, the microneedle 240 can stably penetrate into a scalp without being deformed even with growing resistance just before penetrating into the scalp and transfer an RF to the scalp. The guide part 28 may be formed of a non-metal insulating material, such as rubber, silicon, or plastic, so that the guide part 28 is not influenced by an RF current that is applied to the microneedle 240 functioning as an electrode.

A scalp is covered with a great deal of hair unlike other skin. For this reason, it is difficult to secure the visibility of a treatment portion because. Hair that has been pressed down in a process of bringing the treatment tip into contact with a surface of the scalp for treatment makes the treatment difficult by hindering the penetration of the microneedle into the scalp, or pains may be caused when the microneedle penetrates into the scalp because the microneedle is deformed by the pressed hair.

Furthermore, conventionally, the front ends (apexes) of needles drawn out to the outside have a needle arrangement in which the front ends are arranged on the same plane. Accordingly, unlike in another body portion, in the case of a scalp having curved contour, an insertion deviation (or a penetration depth deviation) may occur between the needles upon treatment. As a result, a treatment effect may be halved or a side effect may occur in severe cases because a skin layer having a desired depth is not accurately targeted.

The treatment tip 20 for a handpiece according to an embodiment adopts a method of improving a scalp environment by simultaneously making the plurality of microneedles penetrate into a treatment target portion and apply electric stimuli to the a treatment target portion, and also has a geometrical shape suitable for treatment for a scalp having a curved surface shape. Accordingly, when treatment is performed on a scalp, the visibility of a treatment target portion can be smoothly secured. Furthermore, when treatment is performed on a scalp, an insertion deviation (or a penetration depth deviation) problem between needles can be solved.

Major components of the treatment tip for a handpiece according to an embodiment are described more specifically.

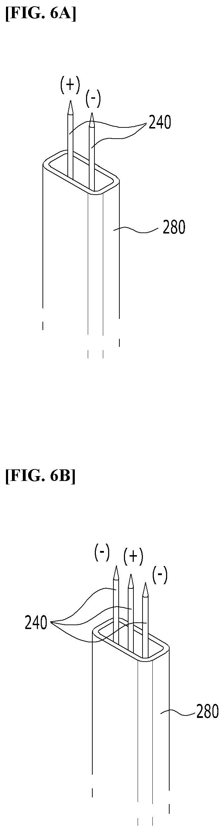

FIGS. 6A and 6B are cross-sectional views of the tubular needle guide for illustrating the state in which the microneedles have been disposed.

Referring to all of FIGS. 6A, 6B and 3 to 5, the plurality of tubular needle guides 280 is disposed in one or more lines in a first direction, but adjacent tubular needle guides 280 may be disposed at predetermined intervals. That is, the treatment tip 20 according to an embodiment may generally have a hair comb shape. Accordingly, it is possible to secure visibility for treatment because disheveled hair at a treatment target portion is tidied up through combing right before the treatment.

In the treatment tip 20 for a handpiece according to an embodiment, some of the plurality of microneedles 240 may be accommodated into the cap part 22 along with the substrate 244. Furthermore, other some of the plurality of microneedles 240 may be divided at least two or more and accommodated in each of the plurality of tubular needle guides 280 as illustrated in FIG. 6A and 6B in the state in which the microneedles 240 have not drawn out. That is, at least two needles 240 may be disposed within one tubular needle guide 280.

Two or more microneedle 240 disposed within one tubular needle guide 280 may include at least one positive electrode needle and at least one negative electrode needle. For example, in a construction in which two microneedles 240 are disposed within one tubular needle guide 280 as illustrated in FIG. 6A, one of the two microneedles may constitute a positive electrode (i.e., a + electrode) to which a (+) voltage is applied. The other of the two microneedles may constitute a negative electrode (a - electrode) to which a (-) voltage is applied.

As another example, in a construction in which three microneedles 240 are disposed in a row in one tubular needle guide 280 as illustrated in FIG. 6B, the three microneedles 240 may include one positive electrode needle and two negative electrode needles or two positive electrode needles and one negative electrode needle. In this case, in the polarity arrangement of the three microneedles 240, the polarity of the microneedle 240 disposed at the center of the polarity arrangement and the polarities of the microneedles 240 disposed at both ends of the polarity arrangement may be arranged to be opposite to each other.

The guide part 28 including the plurality of tubular needle guides 280 is subjected to injection molding along with the cap part 22 through injection molding when the cap part 22 is fabricated. Accordingly, the guide part 28 may be formed in the cap part 22 in a single object form (or an integrated type) as described above. Alternatively, the guide part 28 may be separately fabricated in a process different from a process of fabricating the cap part 22, and may be constructed as a self-assembly (not illustrated) in which the guide part 28 is assembled with the cap part 22 in a separate assembly process.

According to circumstances, only the guide part 28 may be made of a transparent resin material. In this case, the replacement of a deformed microneedle 240 or the disinfection or cleaning of a contaminated microneedle 240 can be timely performed because a surgical operator can directly check the state of the microneedle 240 disposed within the tubular needle guide 280, for example, a deformation or contamination state of the microneedle 240 with his or her own eyes.

The tubular needle guides 280 disposed in the same line, among the plurality of tubular needle guides 280 disposed in one or more lines in the first direction, may be formed to have different lengths. For example, as in the examples of FIGS. 3 to 5, if the plurality of tubular needle guides 280 has been disposed in one line in the first direction, the plurality of tubular needle guides 280 may be formed to have different lengths.

In this case, two or more microneedles 240 disposed within the same tubular needle guide 280 may have the same length, and microneedles 240 disposed within different tubular needle guides 280 may have different lengths. In an embodiment, the length of the microneedle may refer to a length from the substrate 244 to the apex 242 (thin and pointed tip) of each microneedle 240.

For example, a virtual line L1 (refer to FIG. 5) that connects the needle entry and exit ends 282 of tubular needle guides 280 that are disposed in the same line in the first direction may be formed to have predetermined curvature. As another example, the tubular needle guides 280 disposed in the first direction may be formed in the hair comb shape (refer to FIGS. 3 and 5) in which the lengths of the tubular needle guides 280 disposed on the outermost parts on both sides thereof are the longest and the lengths of the tubular needle guides 280 toward the center of the tubular needle guides 280 are gradually reduced.

For reference, the needle entry and exit end 282 refers to the end (i.e., top) of each needle guide 280, and refers to a portion at which a hole through which the apex 242 of each microneedle 240 penetrates or enters or exits when the microneedle 240 disposed within the needle guide 280 is drawn out to the outside of the needle guide 280 or the microneedle 240 drawn out to the outside returns to its original accommodation location (i.e., the standby location for treatment) has been formed.

In an embodiment, the curvature of the virtual line L1 may be determined based on a design in which an average head size of a person by gender and age group and curvature of a surface of the head are considered. According to circumstances, a surgical operator may select a treatment tip suitable for a shape of the head of a patient and perform optimal treatment because the treatment tip is provided in various types in which the curvature of the virtual line L1 is different.

The hair comb shape in which, among the tubular needle guides 280 disposed in one line, the lengths of the tubular needle guides 280 disposed on the outermost parts on both sides thereof are relatively long and the lengths of the tubular needle guides 280 disposed toward the center of the tubular needle guides 280 are gradually reduced is a geometrical shape corresponding to a shape of a scalp surface contour of which is a curved surface. Upon treatment, the needle entry and exit end 282 of the tubular needle guides 280 may fully come into contact with a scalp surface stably and uniformly without being lifted off.

Upon drawing-out operation of the microneedle 240, each microneedle 240 may be drawn out from the needle entry and exit end 282 of a corresponding tubular needle guide 280 in the same length. Furthermore, as described above, the guide part 28 has a geometrical shape corresponding to a shape of a scalp surface contour of which is a curved surface. Accordingly, upon treatment for the scalp, the microneedles 240 drawn out from the needle entry and exit ends 282 in the same length may penetrate into skin tissues having the same depth and transfer an RF to the skin tissues.

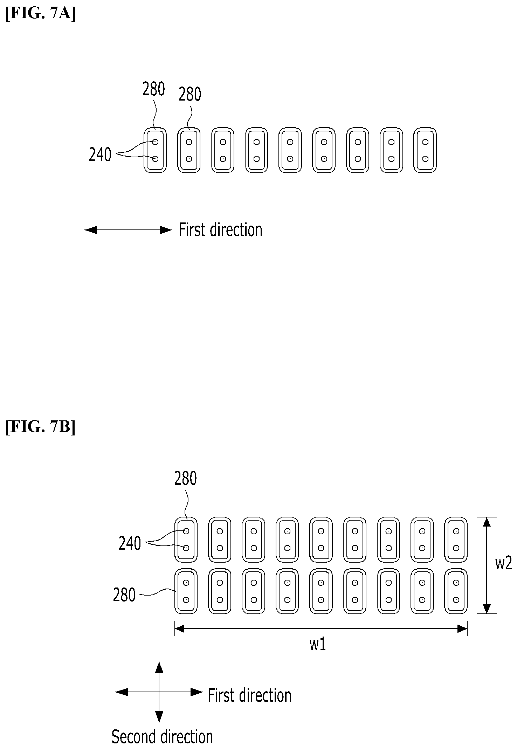

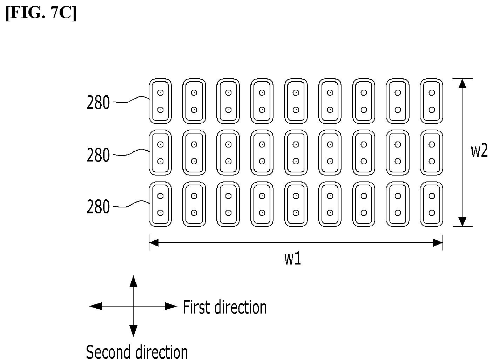

FIGS. 7A to 7C are plan views of the guide part, which illustrates various arrangements of the tubular needle guide. As illustrated in FIG. 7A, the plurality of tubular needle guides 280 may be arranged in one line in the first direction. Alternatively, the plurality of tubular needle guides 280 may be arranged in two lines in rows in the first direction as illustrated in FIG. 7B, or may be arranged in three lines in rows in the first direction as illustrated in FIG. 7C.

Although not illustrated in the drawings, the plurality of tubular needle guides 280 may be arranged in four or more lines in rows in the first direction. However, if the plurality of tubular needle guides 280 is disposed in two or more lines in rows in the first direction, it is preferred that the plurality of tubular needle guides 280 is disposed in a form that is advantageous for the generation of a part for securing visibility for treatment, for example, in a matrix form so that a width w1 in the first direction is greater than a width w2 in a second direction that is orthogonal to the first direction (i.e., w1 > w2).

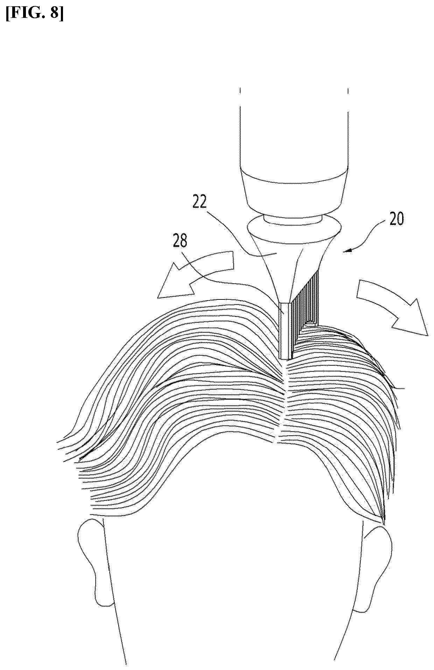

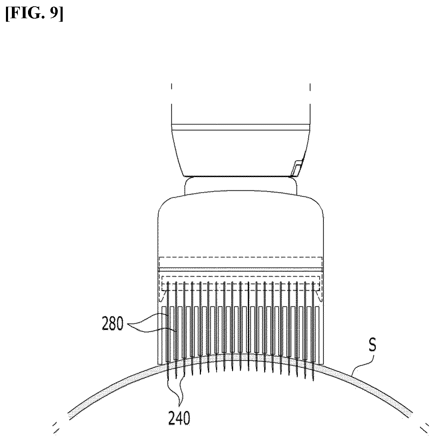

FIGS. 8 and 9 are diagrams illustrating a preparation process for performing invasive RF treatment on a scalp and a form in which invasive RF treatment is performed on a scalp by using the treatment tip according to an embodiment of the present disclosure, respectively.

First, referring to FIG. 8, in the treatment tip 20 for a handpiece according to an embodiment, the guide part 28 provided at the front end of the cap part 22 in an integrated or detachable type has a hair comb shape having predetermined curvature. Accordingly, upon treatment for a scalp, a treatment target portion may be combed by the guide part 28 having the hair comb shape, as in an example of FIG. 8. Accordingly, the accuracy of the treatment can be improved because visibility for treatment on a treatment target portion covered with hair is secured.

Furthermore, the needle entry and exit ends 282 at the front end of the guide part 28 can come into contact with a scalp surface S without being lifted off as illustrated in FIG. 9 because the guide part 28 including the tubular needle guides 280 has a geometrical shape corresponding to a shape of surface contour of a scalp. The microneedles 240 can penetrate into a scalp at the same depth and apply RF stimuli because the microneedles 240 are drawn out from the tubular needle guides 280 in the same length in that state.

The above description is merely a description of the technical spirit of the present disclosure, and those skilled in the art may change and modify the present disclosure in various ways without departing from the essential characteristic of the present disclosure.

Accordingly, the embodiments described in the present disclosure should not be construed as limiting the technical spirit of the present disclosure, but should be construed as describing the technical spirit of the present disclosure. The range of the technical spirit of the present disclosure is not restricted by the embodiments. The range of protection of the present disclosure should be construed based on the following claims, and all of technical spirits within an equivalent range of the present disclosure should be construed as being included in the scope of rights of the present disclosure.

### [Description of reference numerals]

| | | | |
|---|---|---|---|
| 1: | invasive RF treatment device | 10: | handpiece |
| 12: | driving shaft | 20: | treatment tip |
| 22: | cap part | 22a: | upper cap |
| 22b | lower cap | 24: | needle part |
| 26: | elevation pole | 28: | guide part |
| 240 | microneedle | 242: | apex of needle |
| 244 | substrate | 280: | tubular needle guide |
| 282 | needle entry and exit end | | |
| L1: | virtual line that connect apexes of microneedles | | |

## Claims

1. A treatment tip for a handpiece of an invasive radio frequency (RF) treatment device, the treatment tip comprising:
a needle part comprising a plurality of microneedles; and
a guide part comprising a plurality of tubular needle guides in accordance with the plurality of microneedles,
wherein the plurality of tubular needle guides is disposed in one or more lines,
the plurality of microneedles is disposed within the plurality of tubular needle guides, and
a virtual line that connects needle entry and exit ends of the plurality of tubular needle guides disposed in one line has curvature.

2. The treatment tip of claim 1, further comprising:
a cap part detachably installed at a front end of the handpiece; and
an elevation pole having the needle part mounted thereon and movably disposed within the cap part.

3. The treatment tip of claim 2, wherein the guide part is disposed at a front end of the cap part.

4. The treatment tip of claim 3, wherein the guide part is integrally disposed at the front end of the cap part.

5. The treatment tip of claim 3, wherein the guide part is disposed at the front end of the cap part in a form in which the guide part is detachable from the cap part.

6. The treatment tip of claim 1, wherein among the plurality of tubular needle guides disposed in the one line, lengths of the tubular needle guides disposed at outermost parts on both sides thereof are relatively long and lengths of the tubular needle guide are gradually reduced toward a center thereof.

7. The treatment tip of claim 1, wherein two or more microneedles are disposed within one tubular needle guide.

8. The treatment tip of claim 7, wherein the two or more microneedles disposed within the one tubular needle guide have an identical length.

9. The treatment tip of claim 1, wherein upon drawing-out operation of the microneedles, the plurality of microneedles is drawn out from the needle entry and exit ends of corresponding tubular needle guides in an identical length.
